# EUROPEAN PATENT SPECIFICATION

(11) **EP 2 682 458 B1**
(45) Date of publication and mention of the grant of the patent: **23.10.2019**
(21) Application number: 12752625.9
(22) Date of filing: 28.02.2012
(51) Int. Cl.: C12N 5/071, A61K 35/12, A61L 27/00, A61P 9/00, A61P 17/00, A61P 19/02, A61P 27/02, A61L 27/16, A61L 27/38, A61K 35/00, C12N 5/079

(54) **CELL SHEET HAVING HYALURONIC ACID PRODUCTION ABILITY AND METHOD FOR PREPARING THE SAME**
ZELLSCHICHT MIT HYALURONSÄUREPRODUKTIONSFÄHIGKEIT UND VERFAHREN ZU IHRER HERSTELLUNG
FEUILLE DE CULTURE DE CELLULES CAPABLE DE PRODUIRE DE L'ACIDE HYALURONIQUE, ET PROCÉDÉ DE PREPARATION DE CELLE-CI

(30) Priority: 28.02.2011 JP 2011058515
(43) Date of publication of application: 08.01.2014
(73) Proprietor: CellSeed Inc., Tokyo 135-0064 (JP); National University Corporation Hokkaido University, Sapporo-shi, Hokkaido 060-0808 (JP)
(72) Inventor: SUGIYAMA, Hiroaki, Tokyo 135-0064 (JP); SAKAI, Hideaki, Tokyo 135-0064 (JP); SHINOHARA, Yasuro, Sapporo-shi Hokkaido 060-0808 (JP); TAKEGAWA, Yasuhiro, Sapporo-shi Hokkaido 060-0808 (JP)
(74) Representative: Hoffmann Eitle
(86) International application number: PCT/JP2012/054987
(87) International publication number: WO 2012/118097

(56) References cited:
- EP-A1- 0 382 214
- EP-A1- 1 602 383
- EP-A2- 1 970 439
- WO-A1-2004/069295
- WO-A1-2007/148804
- CN-A- 101 643 760
- JP-A- 2007 080 990
- WU S C ET AL: "Enhancement of chondrogenesis of human adipose derived stem cells in a hyaluronan-enriched microenvironment", BIOMATERIALS, ELSEVIER SCIENCE PUBLISHERS BV., BARKING, GB, vol. 31, no. 4, 1 February 2010 (2010-02-01), pages 631-640, XP026762072, ISSN: 0142-9612, DOI: 10.1016/J.BIOMATERIALS.2009.09.089 [retrieved on 2009-10-12]
- WATANABE K ET AL.: 'Development of transplantable genetically modified corneal epithelial cell sheets for gene therapy.' BIOMATERIALS vol. 28, 2007, pages 745 - 749, XP005842205
- KAYO ARAKI ET AL.: 'Gansoshiki Oyobi Koku Nenmaku Johi Yurai Saibo no Saibo Sheet no Sogo Glycome Kaiseki' JOURNAL OF JAPANESE BIOCHEMICAL SOCIETY 19 August 2011, XP008170980

## Description

### TECHNICAL FIELD

The present invention relates to cell sheets useful in the fields of medicine, pharmacy, biochemistry, and biology, methods for preparing the same, and methods for using the same.

### BACKGROUND ART

In recent years, there have been active research and development of various regenerative medicine techniques including transplantation of a patient's cells regenerated *in vitro* into the patient him/herself. The organs to which these techniques are applied have been greatly diversified as exemplified by skin, cornea, kidney, liver, and heart, and the prognosis of these techniques have been significantly improved, so the regenerative medicine has been being established as one of medical technologies. An example is a corneal transplantation; in Japan, the eye bank was organized and corneal transplantation activities started about 50 years ago. However, the number of donors is still small, and it is said that there are about twenty thousand patients per year in Japan alone who need a corneal transplantation but only about a tenth of them, i.e., about two thousand patients, actually undergo this therapy. The current state is that in spite of the presence of the almost established corneal transplantation technology, there is a need for a next medical technology due to the problem of lack of donors. Thus, there was developed a technology to try to culture a patient's normal cells to a desired size and transplant the cultured cells.

Under these circumstances, Patent Document 1 discloses a novel cell culture method in which cells are placed on a cell culture support having a substrate surface coated with a polymer whose upper or lower critical solution temperature in water is 0 to 80°C, the cells are cultured at a temperature either below the upper critical solution temperature or above the lower critical solution temperature, and then the temperature is brought to a temperature either above the upper critical solution temperature or below the lower critical solution temperature, so that the cultured cells are detached without enzymatic treatment. Patent Document 2 discloses that dermal cells are cultured using the above-mentioned temperature-responsive cell culture substrate at a temperature either below the upper critical solution temperature or above the lower critical solution temperature, and then the temperature is brought to a temperature either above the upper critical solution temperature or below the lower critical solution temperature, so that the cultured dermal cells are detached with little damage. The use of such temperature-responsive cell culture substrates has allowed various innovations of conventional culture technologies. Patent Document 3 shows that further developing the above-mentioned technology, specifically preparing a cell sheet composed of parenchymal cells present in hepatic tissues, enables maintenance of the function of hepatic tissue cells over a long period of time, which has not been achieved by conventional technologies. However, there has been so far no investigation at all of a hyaluronic acid production ability.

Meanwhile, liquid chromatography has been used for separation and analysis of solution samples composed of many different types of components. This technique uses a reverse phase column for analysis of hydrophobic molecules and a normal phase column for analysis of hydrophilic molecules. However, since biological samples contain both hydrophobic and hydrophilic molecules, conventional methods for separating and analyzing such biological samples generally require that the respective types of molecules should be analyzed separately using two systems, one equipped with a reverse phase column and the other with a normal phase column, or that hydrophobic molecules alone should be purified with a reverse phase trap column before being analyzed using a reverse phase column, or hydrophilic molecules alone should be purified with a normal phase trap column before being analyzed using a normal phase column; thus, such conventional methods cause analysis procedures to become complicated (refer to Non-patent Documents 1-3). In particular, as regards sugar chain molecules, there is only a limited choice of analysis methods and analytical steps are complicated, so a simpler and more effective analysis method has been long needed.

Under these circumstances, the present inventors have built a technology that makes it possible to minutely examine the sugar chains in glycoproteins or glycopeptides, which were so far difficult to analyze in detail. Among these sugar chains, glycosaminoglycans (GAGs) are known to be polysaccharides that are mainly present in cell membranes or extracellular matrices (ECMs) as protein-linked or non-protein-linked glycans, and which are chemically structured such that the basic disaccharide structure consisting of uronic acid and hexosamine is repeated, and which undergo varying degrees of sulfation. Based on the differences in constitutional disaccharides, glycosaminoglycans are mainly classified into three types: chondroitin sulfate/dermatan sulfate, heparan sulfate/heparin, and hyaluronic acid. Chondroitin sulfate/dermatan sulfate is composed of the disaccharides of glucuronic acid/iduronic acid(β1→)N-acetylgalactosamine, heparan sulfate/heparin is composed of the disaccharides of glucuronic acid/iduronic acid(β1→4)N-acetylglucosamine, and hyaluronic acid is composed of the disaccharides of glucuronic acid(β1→3)N-acetylglucosamine. Since these glycosaminoglycans are further modified by sulfation, they have extremely diversified structures. The glycosaminoglycans are known as important biological substances which, depending on the differences in molecular size and sulfation pattern, have both physicochemical properties derived from characteristic viscoelasticity and biological properties mediated by interactions with various functional proteins. No attempt has been so far made to use these findings for construction of the above-mentioned regenerative medicine techniques.

### CITATION LIST

### PATENT DOCUMENTS

[Patent Document 1] Japanese Unexamined Patent Application Publication No. H02-211865
[Patent Document 2] Japanese Unexamined Patent Application Publication No. H05-192138
[Patent Document 3] Japanese Patent Domestic Re-publication No. 2007-080990

### NON-PATENT DOCUMENTS

[Non-patent Document 1] Anal. Chem., 75, 5628-5637 (2003)
[Non-patent Document 2] Anal. Chem., 76, 6560-6565 (2004)
[Non-patent Document 3] J. Proteome Res., 3, 556-566 (2004)

### SUMMARY OF INVENTION

### TECHNICAL PROBLEM

The present invention has been made with the invention of offering regenerative medicine by designing a more advantageous cell sheet to establish a regenerative medicine technique. In other words, this invention provides a novel cell sheet based on the idea completely different from the prior art, a method for preparing the same, a method for using the same.

### SOLUTION TO PROBLEM

The present inventors, with the use of the sugar chain analysis techniques that were so far constructed by themselves, found that when cells constituting a tissue in a living body are cultured, the cultured cells start actively producing hyaluronic acid (hereinafter also referred to as "HA"). The inventors also found that the HA production ability of the cultured cells significantly increases under such specific conditions that the stem cell content in said cells can be kept high.

More specifically, the present invention provides a cell sheet highly producing hyaluronic acid. This invention also provides a method for preparing a cell sheet, wherein cells are placed on a cell culture support having a surface coated with a polymer which changes a hydration ability in a temperature range of 0-80°C, said cells are cultured in a temperature range where the polymer exhibits a weak hydration ability, and then the temperature of a culture solution is changed to a temperature at which the polymer exhibits a strong hydration ability, so that the cultured cells are detached in the form of sheet, with their hyaluronic acid production ability being increased. The present inventors believe that this invention is a very important invention that cannot be realized without the use of the cell sheet which is a cell construct based on a unique and novel idea in the world.

### ADVANTAGEOUS EFFECTS OF INVENTION

Any cell sheets prepared according to the present invention produce hyaluronic acid with high efficiency. It is expected that the produced hyaluronic acid will act through the transplanted cell sheet on a recipient's body, displaying favorable effects to the recipient's body, such as reinforcement of a stromal tissue, reinforcement of cell-to-cell adhesion, delay in keratinization, and promotion of tissue thickening.

### BRIEF DESCRIPTION OF DRAWINGS

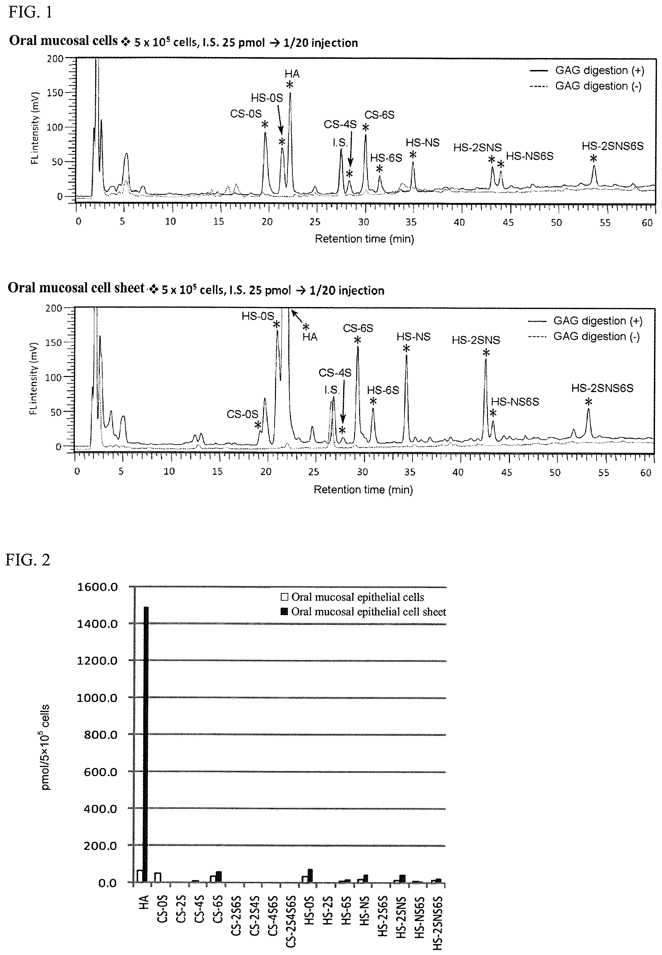

FIG. 1 shows the results of determining the hyaluronic acids (each indicated "HA" in the graphs) produced from the oral mucosal cells and the cell sheet thereof as obtained in Example 1.
FIG. 2 shows the results of comparing the amounts of the hyaluronic acids (each indicated "HA" in the graphs) produced from the oral mucosal cells and the cell sheet thereof as obtained in Example 1.

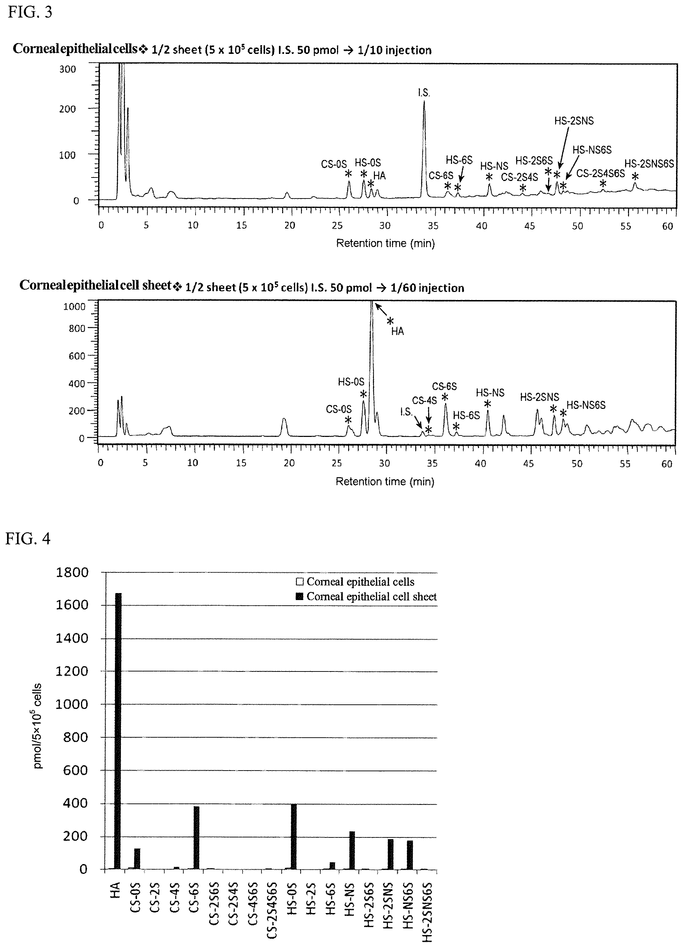

FIG. 3 shows the results of determining the hyaluronic acids (each indicated "HA" in the graphs) produced from the corneal epithelial cells and the cell sheet thereof as obtained in Example 2.
FIG. 4 shows the results of comparing the amounts of the hyaluronic acids (each indicated "HA" in the graphs) produced from the corneal epithelial cells and the cell sheet thereof as obtained in Example 2.

### DESCRIPTION OF EMBODIMENTS

The cell sheet as set forth in the present invention is such that produces hyaluronic acid with high efficiency. The method for preparing the cell sheet is not particularly limited as long as it is a method by which cells cultured on a surface of a substrate can be finally harvested in the form of sheet, and examples include a method in which a surface of a substrate is coated with a cell adhesive protein or peptide, a synthetic polymer, and/or a temperature-responsive polymer, cells are cultured on the coated surface, and a sheet of the cultured cells is detached. Here, the substrate having a temperature-responsive polymer coated thereon is taken as an example. Cells placed on a cell culture support having a surface coated with a polymer which changes a hydration ability in a temperature range of 0-80°C can be cultured in a temperature range where the polymer exhibits a weak hydration ability. The preferred temperature is 37°C, a temperature at which cells are usually cultured. The temperature-responsive polymer used in the present invention may be either a homopolymer or a copolymer. Examples of these polymers include polymers described in Japanese Unexamined Patent Application Publication No. H02-211865. Specifically, such polymers are typically prepared by homopolymerization or copolymerization of the following monomers. Examples of monomers that can be used include (meth)acrylamide compounds, N-(or N,N-di)alkyl-substituted (meth)acrylamide derivatives, and vinyl ether derivatives. Copolymers can be made of any two or more of the above-mentioned monomers. Alternatively, they may be prepared by copolymerization of any of the above-mentioned monomers with any other monomer than said monomers, or by graft polymerization or copolymerization of polymers. A mixture of polymers or copolymers may also be used. Such polymers may also be crosslinked as long as their inherent properties are not impaired. Considering that separation takes place at a temperature ranging from 5 to 50°C since it is cells that are to be cultured and detached on the temperature-responsive polymer, the polymer can be exemplified by poly-N-n-propylacrylamide (homopolymer's lower critical solution temperature, 21°C), poly-N-n-propylmethacrylamide (ditto, 27°C), poly-N-isopropylacrylamide (ditto, 32°C), poly-N-isopropylmethacrylamide (ditto, 43°C), poly-N-cyclopropylacrylamide (ditto, 45°C), poly-N-ethoxyethylacrylamide (ditto, ≈35°C), poly-N-ethoxyethylmethacrylamide (ditto, ≈45°C), poly-N-tetrahydrofurfurylacrylamide (ditto, ≈28°C), poly-N-tetrahydrofurfurylmethacrylamide (ditto, ≈35°C), poly-N,N-ethylmethylacrylamide (ditto, 56°C), and poly-N,N-diethylacrylamide (ditto, 32°C). Exemplary monomers and polymers for copolymerization that are used in this invention include, but are not particularly limited to, hydrated polymers such as polyacrylamide, poly-N,N-diethylacrylamide, poly-N,N-dimethylacrylamide, polyethylene oxide, polyacrylic acid and a salt thereof, and hydrated polymers such as polyhydroxyethyl methacrylate, polyhydroxyethyl acrylate, polyvinyl alcohol, polyvinyl pyrrolidone, cellulose, and carboxymethyl cellulose.

The method used for coating the surface of the substrate with such a polymer as mentioned above in the present invention is not particularly limited, and the coating can typically be performed by subjecting the substrate and said monomer or polymer to electron beam (EB) irradiation, γ-ray irradiation, ultraviolet ray irradiation, plasma treatment, corona treatment, or organic polymerization reaction, or by physical adsorption through spread coating, kneading or the like. The coating amount of the temperature-responsive polymer on the surface of the culture substrate can be in the range of 1.1 to 2.3 µg/cm², preferably 1.4 to 1.9 µg/cm² and more preferably 1.5 to 1.8 µg/cm². A coating amount lower than 1.1 µg/cm² is undesirable since it makes it difficult for cells to be detached from the polymer even if a stimulus is applied, leading to a significant deterioration in work efficiency. Also, a coating amount higher than 2.3 µg/cm² makes it difficult for cells to adhere to the area of interest, preventing them from adhering adequately. In such as case, if cell adhesive proteins are further applied onto the temperature-responsive polymer coating layer, the coating amount of the temperature-responsive polymer on the substrate surface can be higher than 2.3 µg/cm², but this coating amount is advantageously not higher than 9.0 µg/cm², preferably not higher than 8.0 µg/cm², and more preferably not higher than 7.0 µg/cm². A coating amount higher than 9.0 µg/cm² is undesirable since it makes it difficult for cells to adhere even if such cell adhesive proteins are further applied onto the temperature-responsive polymer coating layer. The cell adhesive proteins can be of any type without particular limitation, and may be any one of collagen, laminin, laminin 5, fibronectin, matrigel and the like, or a mixture of two or more of them. The cell adhesive proteins can be applied according to any conventional protocol, and are commonly applied by coating the substrate surface with the cell adhesive proteins in aqueous solution, removing the aqueous solution, and performing rinsing. This invention is a technique using a temperature-responsive culture dish with the aim of putting a cell sheet itself to use to the extent possible. Therefore, an extremely large coating amount of the cell adhesive proteins on the temperature-responsive polymer layer is undesirable. The coating amounts of the temperature-responsive polymer and the cell adhesive proteins can be measured according to any conventional protocol; for example, they may be measured by directly measuring the area to which cells adhere using the FT-IR-ATR method, or by a method in which a polymer that has been labeled beforehand is immobilized using the same procedure and the amount of the labeled polymer immobilized on the area to which cells adhere is determined to estimate the coating amounts, and any other methods may also be used.

In the present invention, a sheet of cultured cells can be detached and harvested from the temperature-responsive substrate by bringing the temperature of the culture substrate to which the cultured cells adhere to a temperature either above the upper critical solution temperature or below the lower critical solution temperature of the coating polymer on the culture substrate. The detachment may be performed in a culture solution or any other isotonic solution -- the solution to be used can be selected depending on the purpose. For the purpose of detaching and harvesting the cells more quickly and efficiently, any various methods including lightly tapping or shaking the substrate, or stirring a culture medium with a pipette may be used alone or in combination. The culture conditions other then temperature may be pursuant to any conventional protocol and are not particularly limited. For example, the medium to be used may be a medium having a known serum such as fetal bovine serum (FCS) added thereto or a serum free medium having no such serum added thereto.

The above-mentioned detachment and harvesting method is now described by referring to the case of using poly(N-isopropylacrylamide) as the temperature-responsive polymer. Poly(N-isopropylacrylamide) is known as a polymer having a lower critical solution temperature of 31°C. When in a free state, it dehydrates at a temperature higher than 31°C in water, and the polymer chains aggregate to cause cloudiness. In contrast, at a temperature of 31°C or lower, the polymer chains hydrate to become dissolved in water. In the present invention, a coating of such a polymer is applied and immobilized onto the surface of a substrate such as a culture dish. Accordingly, at a temperature higher than 31°C, the polymer on the substrate surface dehydrates as mentioned above, but since the coating of the polymer chains has been applied and immobilized on the substrate surface, the latter becomes hydrophobic. On the other hand, at a temperature of 31°C or lower, the polymer on the substrate surface hydrates, whereupon the substrate surface becomes hydrophilic because the polymer chains has been applied and immobilized onto the substrate surface. The hydrophobic surface is an adequate surface for cells to adhere and grow, whereas the hydrophilic surface is such a surface that prevents cells from adhering; thus, cultured cells or cell sheets can be detached simply by cooling.

The substrate to be coated can be any ordinary substrate that can be given a shape, including compounds that are commonly used in cell culture, such as glass, modified glass, polystyrene, and polymethylmethacrylate. Any other polymeric compounds than the above-listed ones as well as all kinds of ceramics can also be used.

The form of the culture substrate in the present invention is not particularly limited, and examples include forms like a dish, a multiplate, a flask, a cell insert, and a flat membrane.

In the present invention, when the substrate having a temperature-responsive polymer coated thereon is used, a cultured cell sheet is not damaged by a protease as typified by dispase or trypsin, so that the cell sheet as detached from the substrate has cell adhesive proteins and the cells as detached in the form of sheet maintain a cell-cell desmosome structure to some extent. This allows the cell sheet as transplanted to graft to a diseased tissue satisfactorily, thereby enabling efficient transplantation. It is generally know that the proteinase dispase can detach cells with 10-40% of a cell-cell desmosome structure being kept intact. However, this proteinase breaks down most of basement membrane-like proteins formed between cells and a substrate, so that the resultant cell sheet will have weak strength. In contrast, the cell sheet of the present invention has kept intact at least 60% of both a desmosome structure and basement membrane-like proteins and thus can provide such various effects as mentioned above.

The cells to be used in the present invention are not particularly limited as long as they are capable of producing hyaluronic acid; for example, they may be any one type of cells among oral mucosal cells, limbal epithelial cells, corneal epithelial cells, conjunctival epithelial cells, chondrocytes, synovial cells, epidermal keratinocytes, smooth muscle cells, vascular endothelial cells, fibroblasts and mammary epithelial cells, or a mixture of two or more types thereof. The respective types of cells may also have their stem cells contained therein. Among these types of cells, oral mucosal cells as well as epithelial cells such as limbal epithelial cells, corneal epithelial cells, conjunctival epithelial cells, and epidermal keratinocytes actively produce hyaluronic acid, so they are advantageously used alone or in admixture with other types of cells. It is advantageous to use stem cells because they may greatly contribute to the production of hyaluronic acid, which is an object of this invention. Further, the stem cells may be mixed with other cells. The above-mentioned types of cells can be cultured according to any conventional protocol in a suitable medium at a suitable seeding density for a suitable culture period for each type of cells, but in order to increase the hyaluronic acid production ability of the cells, it is important to culture them so as not to reduce their stem cell content. The culture method for attaining this result is also not particularly limited, and examples include a method in which feeder cells are used in combination for culture of epithelial cells. In this invention, the stem cell content in the cultured cells is at least 0.5%, preferably at least 1%, more preferably at least 2%, still more preferably at least 5%, and most preferably at least 8%.

The cells to be used in the present invention may be exemplified by cells collected directly from a biological tissue, cells collected directly from said tissue and differentiated as in a culture system, or a cell line, but the types of the cells are not limited at all. The origins of the cells are not particularly limited, and examples include human, rat, mouse, guinea pig, marmoset, rabbit, dog, cat, sheep, pig or chimpanzee, or those animals with immunodeficiency. In the case where the cells of this invention are used for treatment of humans, cells derived human, it is preferred to use human-, pig-, or chimpanzee-derived cells.

In the present invention, the cells to be seeded must be dissociated by enzymatic treatment. The enzymatic treatment can be performed according to any conventional protocol without particular limitation. The number of the cells to be seeded for culture varies with the animal species from which the cells to be used originate, but is generally advantageously in the range from 0.4×10⁶ to 2.5×10⁶ cells/cm², preferably in the range from 0.5×10⁶ to 2.1×10⁶ cells/cm², and more preferably in the range from 0.6×10⁶ to 1.7×10⁶ cells/cm². A seeding density lower than 0.4×10⁶ cells/cm² is undesirable for working of this invention because it causes deterioration of cell proliferation, leading to a decrease in the expression of the functions of the resultant cell sheet.

The cell sheet of the present invention can be prepared in such a manner as described above. In this invention, multiple layers of the cell sheet can also be stratified. The number of layers to be stratified is not particularly limited, and the stratifying frequency is advantageously 10 times or less, preferably 8 times or less, and more preferably 4 times or less. Stratifying cell sheet layers is desirable since this results in an increase in cell density per unit sheet area, enhancing the functions of the cell sheet.

The cell sheet stack or the cell sheet laminate to be used in the present invention may also be a stack or laminate of a combination of sheets composed of different types of cells, as exemplified by cell sheets that are composed of vascular endothelial cells, vascular endothelial precursor cells, or the like and which are intended for introducing blood vessels into the cell sheets, or cell sheets that are composed of chondrocytes and which are intended for immunoisolating the cell sheets. It is desirable to use two or more different types of cells because interaction between such different types of cells yields a cell sheet with higher activity. The position, order and frequency of stratifying cell sheets are not particularly limited, and can be varied as appropriate depending on the tissue to be coated or replaced, typically through the use of a cell sheet having strong adhesion. The stratifying frequency is advantageously 10 times or less, preferably 8 times or less, and more preferably 4 times or less. In the case where vascular endothelial cells are selected, the problem with stacking can be prevented by constructing a vascular network in the cell sheet stack or the cell sheet laminate. The method for constructing a vascular network is not particularly limited, and may be exemplified by a method in which vascular endothelial cells are incorporated in a cell sheet stack or a cell sheet laminate beforehand, a method in which a vascular endothelial cell sheet is stacked in the process of preparing a cell sheet stack or a cell sheet laminate, or a method in which a cell sheet stack or a cell sheet laminate is implanted in a living body to construct a vascular network.

The method for preparing a cell sheet stack or a cell sheet laminate according to the present invention is also not particularly limited; for example, the cell sheet stack or the cell sheet laminate can be prepared by detaching cultured cells in the form of sheet and placing one sheet of the cultured cells on another using a cultured cell transfer tool dependent on the need. In the process, the temperature of a medium is not particularly limited as long as it is below the upper critical solution temperature, if any, of the above-mentioned polymer applied to the surface of a culture substrate or it is above the lower critical solution temperature, if any, of said polymer. Needless to say, it is inappropriate to perform culture in a low temperature range where no cultured cells grow or in a high temperature range where cultured cells die. The culture conditions other then temperature may be pursuant to any conventional protocol and are not particularly limited. For example, the medium to be used may be a medium supplemented with a known serum such as fetal bovine serum (FCS) or a serum free medium supplemented with no such serum. The culture cell transfer tool to be used is not particularly limited as long as it can pick up a detached cell sheet; and examples include membranes, plates, and sponges as exemplified by porous membranes, papers, and rubbers. Alternatively, any tool that includes a handle and has a membrane, plate or sponge (e.g., porous membrane, paper or rubber) attached thereto may be used to facilitate a stratifying process.

In the present invention, for the purpose of stabilizing the functions of the cell sheet, the sheet can be coated with a synthetic polymer or a natural polymer or can be microencapsulated according to any conventional protocol. The method for polymer coating, the method for microencapsulation, and the materials to be used in said methods are not limited at all, and a common practice is to provide a material such as polyvinyl alcohol, urethane, cellulose and derivatives thereof, chitin, chitosan, collagen, polyvinylidene difluoride (PVDF), or silicone in the form of membrane, porous membrane, nonwoven fabric, or fabric and bring the cell sheet into contact with the material before use.

Thus, the cell sheet stack or the cell sheet laminate of the present invention has high strength with few structural defects for the following reasons: the cultured cell sheets detached from a cell culture substrate coated with a temperature-responsive polymer and picked up optionally using a cultured cell transfer tool are not damaged by a protease as typified by dispase or trypsin during culture; basement membrane-like proteins formed between the cells and the substrate during culture are also not enzymatically broken down; and the desmosome structure between the cells is maintained.

The carrier to be used for adhering cell sheets is a construct for holding the cells of the present invention, and examples of the carrier that can be used include polymeric membranes, constructs molded from polymeric membranes, and metallic jigs. Taking the case of using a polymer as the material of a carrier, specific examples of that material include polyvinylidene difluoride (PVDF), polypropylene, polyethylene, cellulose and derivatives thereof, paper, chitin, chitosan, collagen, urethane, and gelatin. The form of the carrier is not particularly limited.

The cell sheet prepared according to the present invention can be transplanted onto a given site in a living body. The transplantation site can be any site in the living body without particular limitation, and may be exemplified by eyeball, skin tissue, omentum, intra-abdominal tissue, subperitoneal tissue, joint, blood vessel wall, muscle, fascial tissue, and cardiac valve tissue. Omentum is particularly preferred since it is rich in blood vessels and easy to transplant. The transplantation site may or may not have blood vessels induced therein beforehand -- this is not a particularly critical matter. Also, the method for inducing blood vessels is not particularly limited, and may be exemplified by a method in which the vascular growth factor FGF is embedded in a microsphere and the microsphere is brought to act on a living body for 8-10 days while the makeup, size and injection range of the microsphere is changed, and a method in which a polyethylene terephthalate mesh is cut to a given size and shaped into a bag, a FGF dissolved in a high-concentration agarose solution is placed into the bag, and after being implanted for 8-10 days, the bag is removed, whereupon a space having blood vessels induced therein is created.

The cell sheet to be transplanted according to the present invention shows an extremely high engraftment because it retains basement membrane-like proteins. The number of cells to be transplanted can be varied depending on the purpose, and when the cells to be transplanted has the form of sheet, the activity levels of various cellular functions can be changed by changing the size and shape of the cell sheet.

If the cell sheet as set forth in the present invention is applied to a human recipient, it is expected that the transplanted cell sheet will express hyaluronic acid in the recipient's body over a long period of time, and that the expressed hyaluronic acid will act through the transplanted cell sheet on the recipient body, displaying favorable effects to the recipient's body, such as reinforcement of a stromal tissue, reinforcement of cell-to-cell adhesion, delay in keratinization, and promotion of tissue thickening.

The method for quantifying the hyaluronic acid produced by the cells cultured in the present invention is not particularly limited, and may be any one of liquid chromatography (HPLC), mass spectrometry (MS), and MS/MS, or a combination of two or more of them. It is particularly advantageous to determine at least two types of glycosaminoglycans by HPLC using a column that uses a stationary phase having anion- and cation-exchange groups, since this method can also obtain the information of other sugar chains present on the cell surface. The stationary phase having anion- and cation-exchange groups may, for example, consists of a core composed of an organic resin and many zwitterionic non-aromatic groups covalently bonded on the surface of the core. Such a stationary phase is typically described in Japanese Patent Domestic Publication No. JP 2002-529714. This phase is more specifically described below.

The "organic resin" as referred to in connection with the stationary phase refers to a synthetic or naturally-derived organic polymer or copolymer comprising mono- or oligovinyl monomer units such as styrene and its adduct derivatives, acrylic acid or methacrylic acid, alkyl acrylates and alkyl methacrylates, hydroxyalkyl acrylates and hydroxyalkyl methacrylates, acrylamides and methacrylamides, vinylpyridine and its adduct derivatives, divinylbenzene, divinylpyridine, alkylene diacrylate, alkylene dimethacrylate, oligoethylene glycol diacrylate and oligoethylene glycol dimethacrylate having up to 5 ethylene glycol repeating units, alkylene bis(acrylamides), piperidine bis(acrylamide), trimethylolpropane triacrylate, trimethylolpropane trimethacrylate, pentaerythriol triacrylate and pentaerythriol tetraacrylate, and mixtures thereof. Alternatively it can be a carbohydrate polymer such as agarose, cellulose, dextran, chitosan, or a crosslinked derivative thereof. Such an organic resin constitutes the core of the stationary phase.

The "zwitterionic non-aromatic group" refers to an added ionic functional non-aromatic group that serves as a pendant group having both a positive and a negative charge and which, as a result, has no net charge under the main conditions during use. Such groups are those which exist as monomeric units directly attached to a backbone polymer, or as linear or crosslinked polymeric or copolymeric layers composed at least partially of non-aromatic zwitterionic monomer units so that each of the pendant groups attached to a backbone polymer has a polyvalent zwitterionic non-aromatic group.

Zwitterionic non-aromatic groups are classified as "strong" or "weak" depending on whether ionic non-aromatic groups are capable of undergoing dissociation or protonation equilibria in an aqueous pH range applicable to separation of biological macromolecules. Examples of strong ionic non-aromatic groups include sulfonic acid and quaternary ammonium groups, whereas examples of weak ionic groups include carboxylic groups and alkylamine or hydroxyalkylamine. Examples of strong/strong zwitterionic non-aromatic groups include sulfoalkylammoniobetaines, sulfoalkylarsenobetaines, phosphonoalkylammoniobetaines, and phosphonoalkylarsenobetaines. Weak/strong non-aromatic zwitterionic groups, which each comprise one strong charge and one weak charge, will have no net charge if they are zwitterionic, or will be positive or negative depending on whether weak ionic groups are protonated, dissociated as anionic groups, or in an intermediate state. Weak/weak zwitterionic groups can be exemplified by a) α-amino acids each having a neutral side chain attached to an α-amino group by alkyl coupling, or b) amino acids which are α-protected, attached through reactive groups on their side chains to form an uncharged covalent bond, and then subjected to deprotection of their α-amino groups; in both cases, amphoteric pendant groups are formed that are capable of possessing positive, negative or no net charge depending on the pH in the surroundings. In the absence of a net charge, those groups which are dissociative or capable of being protonated are in equilibrium between the zwitterionic form having a pair of opposite charges and the neutral/neutral, non-zwitterionic form.

Examples of a vinylic monomer having a strong-strong non-aromatic zwitterionic group covalently attached thereto include 3-(2-acrylamido-2-methylpropanedimethylammonio)-1-butanesulfonate, 4-(2-acrylamido-2-methylpropanedimethylammonio)-1-butanesulfonate, 2-methacryloxyethyl phosphorylcholine, 4-[(2-acrylamido-2-methylpropyl)dimethylammonio]butanoate, and 3-[N-decyl,N-(2-methacryloxyethyl),N-methyl]ammoniopropanesulfonate.

Polymerizable monomers forming such non-aromatic zwitterionic groups are hereinafter referred to as "zwitterionic monomers". Likewise, porous selective sorbents forming non-aromatic zwitterionic groups are hereinafter referred to as "porous zwitterionic sorbents".

It is not important which one to choose, polymer or copolymer, so a lot of different types of organic resins can be used. The functional zwitterionic groups densely distributed on the resin surface provide an electrostatic barrier which shields the underlying separation carrier from interacting with biological macromolecules. Thus, the main role of the organic resin is to act as a stable separation carrier for the non-aromatic zwitterionic groups. Therefore, which one to choose, polymer or copolymer, is not important as long as the selected organic resin has, on its surface, reactive groups that can be attached using a chemical method. Accordingly, the non-aromatic zwitterionic pendant groups can be attached to many different types of organic resins.

Specifically, the non-aromatic zwitterionic monomer can be included as part of the monomers constituting the selective adsorption/separation carrier made of the organic resin, so that a porous zwitterionic sorbent can be provided.

The selective adsorption/separation carrier is porous. More specifically, the pore diameter can be in the range from 5 to 50 nm, preferably from 7 to 40 nm, in order to provide a bulk flow channel for an eluting solution.

The zwitterionic non-aromatic groups are bonded to the surface of the separation carrier typically by graft polymerization of monomers comprising the non-aromatic zwitterionic groups. Specifically, the zwitterionic non-aromatic groups can also constitute the whole structure of the selective adsorption/separation carrier by polymerizing the monomers comprising the non-aromatic zwitterionic groups together with crosslinking monomers. More specifically, the zwitterionic non-aromatic groups are bonded to the separation carrier by activation of alkyl groups, and are subsequently reacted with ω-dialkylaminoalkylsulfonic acid to form non-aromatic zwitterionic groups on the separation carrier. With the use of a reaction method known in the art, dialkyl amines (wherein one or both of the alkyl substituents optionally contains a hydroxyl group) are incorporated onto the appropriately activated selective adsorption/separation carrier and then alkyl sulfones are reacted to thereby obtain non-aromatic zwitterionic pendant groups attached to the carrier surface. In a particular embodiment, the selective adsorption/separation carrier is a monolithic porous polymer. The term "zwitterionic non-aromatic group" refers to a functional group attached to the organic resin separation carrier as a single identifiable pendant group, said functional group being characterized by having both a negative and a positive ionic charge, and being bonded onto the organic resin separation carrier directly, or through a reaction for masking the functional group present on the organic resin separation carrier which has been activated, or by polymerizing a monomer containing the functional group having the above-mentioned properties.

The selective adsorption/separation carrier may also be such that is activated by its organic resin surface bearing appropriate reactive functional groups. Such functional groups are exemplified by epoxy groups, alkyl halide groups such as alkyl chloride groups and alkyl bromide groups, and other groups that are capable of alkylating the amino group in aminoalkylsulfonic acid so that said reaction can result in formation of zwitterionic non-aromatic groups covalently bonded on the selective adsorption/separation carrier.

A specific example is the case where the zwitterionic groups resulting from the above-mentioned reaction are ω-sulfoalkyl-trialkylammonium(sulfobetaine) groups -- in this case, at least one of the alkyl derivative groups in the ammonia group may be covalently bonded to the selective adsorption/separation carrier so that one or both of the alkyl groups can carry a hydroxyl group.

The column using a stationary phase having a positive and a negative ion exchange group can be a ZIC (zwitterionic ion chromatography) column as mentioned above, which is commercially available. The ZIC column has been traditionally used for separation of inorganic positive and negative ions and small organic molecular ions, but the present inventors, after conducting intensive studies, surprisingly found that said column can easily separate multiple high-molecular-weight glycosaminoglycans.

The above-mentioned column is fed with an undermentioned sample containing disaccharide units of glycosaminoglycans and then with an organic solvent or a mixture of an organic solvent and water or an aqueous solution, whereby separation of the molecules of interest can be conducted.

Examples of the organic solvent that can be used include acetonitrile, tetrahydrofuran, acetone, dioxane, pyridine, methanol, ethanol, 1-propanol, and 2-propanol, with acetonitrile and methanol being particularly preferred. Such organic solvents can be used alone or in combination of two or more of them.

The organic solvent may be used in combination with water or an aqueous solution. In the case of using the aqueous solution, the salt concentration and/or pH thereof can be adjusted. If the aqueous solution contains a salt, examples of the salt that can be used include ammonium acetate, ammonium carbonate, sodium carbonate, and sodium phosphate. In particular, when a mass spectrometer is used as a detector, volatile salts such as ammonium acetate and ammonium carbonate are preferably used. Such salts can be used alone or in combination of two or more of them.

In the mixture of the organic solvent and water or an aqueous solution, the concentration of the organic solvent can be for example 40% or higher. Alternatively, the organic solvent may also be used at a concentration of 100%. The mixture of the organic solvent and water or an aqueous solution, or the organic solvent, is fed to the column to separate the molecules of interest. The organic solvent concentration may be varied sequentially or intermittently in the course of a separation analysis time (a gradient method commonly used in liquid chromatography) to thereby improve the efficiency in separation analysis.

The separation efficiency (and separation time and ability) can be improved by optimizing the salt concentration and/or pH (as well as optionally temperature) of the solution. The efficiency in separation analysis can also be improved by varying the salt concentration and/or pH sequentially or intermittently in the course of a separation analysis time (a gradient method). In the present method, the molecules eluting from the column or the analyzed molecules can be recycled to the same column at least once. If a single passage through the column cannot achieve sufficient separation, an eluate can be recycled to the same column at least once. Recycling can promote the separation of two or more types of components that are not separated sufficiently.

The present method is characterized by analyzing at least two or more types of glycosaminoglycans at the same time by high performance liquid chromatography using the column that uses the thus-obtained stationary phase having positive and negative ion exchange groups. The glycosaminoglycans of interest are not particularly limited and may be exemplified by compounds composed of chondroitin sulfate or dermatan sulfate, compounds composed of heparan sulfate or heparin, hyaluronic acid compounds, and keratan sulfates. In the present method, the glycosaminoglycans are digested into their constitutional disaccharides before being analyzed. In the process of determining disaccharides derived from chondroitin sulfate and dermatan sulfate (CS and DS) or disaccharides derived from heparan sulfate and heparin (HS and HP), if glycosaminoglycans are digested into disaccharides using an enzyme that cleaves both CS and DS, as exemplified by Chondroitinase ABC, or an enzyme that cleaves both HS and HP, it will be then impossible to distinguish between the disaccharides originally constituting CS and DS, respectively (in terms of quantity and quality) or between the disaccharides originally constituting HS and HP, respectively (in terms of quantity and quality). However, changing the type of a glycosaminoglycan-digesting enzyme makes it possible to distinguish between the respective disaccharides constituting CS and DS or between the respective disaccharides constituting HS and HP, in terms of quantity and quality. The method for distinction is not limited at all; for example, the results of disaccharide analysis using Chondroitinase ABC mentioned above (an enzyme that cleaves both CS and DS) are checked against those using Chondroitinase AC-I Flavo or Chondroitinase AC-II Arthro (an enzyme that does not cleave DS but cleaves CS only) and those using Chondroitinase B (an enzyme that does not cleave CS but cleaves DS only) to thereby allow investigation of the quantity and quality of the disaccharides constituting CS and the disaccharides constituting DS. The same method applied to HS and HP. The present method enables separation and analysis which have been very complicated according to the prior art to be performed much more simply and efficiently. The present method is for analyzing two or more types of glycosaminoglycans at the same time, and the types and their number of glycosaminoglycans are not particularly limited -- three or four types of them may be subjected to analysis. The molecular weight of the glycosaminoglycan molecules of interest is also not particularly limited; for example, those molecules having molecular weights between 100 and several millions are subjected to analysis.

The present method requires that the above-mentioned test substances glycosaminoglycans be digested into their constitutional disaccharide units, and the disaccharides be captured by glycoblotting, liberated by acid treatment, and then fluorescently labeled by reductive amination before being analyzed. The method for digesting glycosaminoglycans into their constitutional disaccharide units is not particularly limited, and a microorganism-derived enzyme such as Chondroitinase ABC (Proteus vulgaris), Chondroitinase AC-I Flavo (Flavobacterium heparinum), Chondroitinase AC-II Arthro (Arthrobacter aurescens), Chondroitinase B (Flavobacterium heparinum), Heparinase (Flavobacterium heparinum), Heparitinase (Flavobacterium heparinum), Hyaluronidase SD (Streptococcus dysgalactiae), Keratanase (Pseudomonas sp.), or Keratanase II (Bacillus sp.) is commonly used. Conditions such as enzyme concentration, substrate concentration, treatment temperature, and treatment time may be selected according to any conventional protocol and are not particularly limited. The resulting disaccharides may optionally be purified using filtration, gel filtration, an adsorption column or the like without any problem, and the treatment conditions for the purification are also not limited at all.

The present method requires that the resulting constitutional disaccharides units of glycosaminoglycans be fluorescently labeled. The steps to be taken until fluorescent labeling are not limited at all; for example, the disaccharides may be fluorescently labeled directly, or the disaccharides may be immobilized on the surface of a carrier having hydrazide groups by glycoblotting, liberated from the carrier by acid treatment to render them as sugar chains having reducing terminals (hemiacetal structure), and then fluorescently labeled by reductively aminating them with compounds having amino groups to open the rings at the reducing terminals of the sugar chains. The term "glycoblotting" refers to a sugar chain purification method characterized in that polymer beads having hydrazide groups and the reducing terminals of sugar chains are linked together in a sugar chain-specific manner through hydrazone formation, and which is a technique for purifying sugar chains or sugar chain derivatives from a biological sample with simple operations. In order to perform this method, the surface of a carrier having hydrazide groups, aminooxy groups or the like is commonly used, but the carrier may also be a product preparing according to Japanese Patent Domestic Re-publication No. 2008-018170 or may be BlotGlyco (Sumitomo Bakelite) which is commercially available. In order to effect capture, the column may be packed with the above-mentioned carrier and then a liquid having disaccharides dissolved therein may be passed through the column, or a carrier may be injected into a disaccharide solution, or alternatively, the disaccharide solution may be continuously charged into a reaction vessel preliminary packed with carrier and then be stirred. The capture reaction conditions are not particularly limited.

In the present method, the disaccharides captured by the thus-obtained carrier are liberated by acid treatment. The acid to be used in the treatment may be exemplified by acetic acid, trifluoroacetic acid, and hydrochloric acid but is not particularly limited. Various treatment conditions such as acid concentration, treatment temperature, and treatment time may be selected according to any conventional protocol and are not particularly limited.

The present method requires the disaccharides treated with acid be reductively aminated. The reducing agent to be used in the process may be exemplified by sodium cyanoborohydride, borane-pyridine complex, and picoline borane but is not particularly limited. Various treatment conditions such as reducing agent concentration, treatment temperature, and treatment time may be selected according to any conventional protocol and are not particularly limited. The fluorescent-labeling agent to be used in the process may be exemplified by 2-aminobenzamide, 2-aminopyridine, 2-aminoacridone, BIDIPY hydrazide, and 2-aminobenzhydrazide but is not particularly limited. An UV-labeling agent such as PMP (1-phenyl-3-methyl-5-pyrazolone) may also be used instead of the fluorescent-labeling agent. Various treatment conditions such as fluorescent-/UV-labeling agent concentration, treatment temperature, and treatment time may be selected according to any conventional protocol and are not particularly limited. Thus, the disaccharides used in the present method will be fluorescent-/UV-labeled compounds.

According to the present method, as long as compounds obtained by digesting glycosaminoglycans originating from a living body-derived sample into disaccharides and fluorescently labeling the disaccharides are used as samples for analysis, multiple types of glycosaminoglycans can be both separated and analyzed in a single run by high performance liquid chromatography using a zwitterionic column.

In the process of analyzing separated molecules, any appropriate method can be used depending on the type of the molecules of interest; for example, mass spectrometry (MS) and/or nuclear magnetic resonance (NMR) analysis can be used. Besides these analysis methods, an ultraviolet (UV) absorptiometer, a evaporative light-scattering (ELS) detector, an electrochemical detector, or the like can also be used.

In the present method, the molecules eluting from the column or the analyzed molecules can be further fractionated. Fractionation of the molecules of interest makes it possible to put the fractionated molecules to further use. The fractionation can be performed typically using a fraction collector.

### EXAMPLES

The present invention will be described below in more detail by way of Examples, but these Examples do not limit the invention at all.

### [Example 1]

Using a rabbit (New Zealand White; Charles River Laboratories Japan) as a model and assuming that the model suffers from a loss of the cornea, oral mucosal epithelial cells were prepared from the rabbit. One fragment of 3-5 mm in diameter was collected from the buccal mucosa and treated with dispase (1000 U, 4°C, 4 h) and then with trypsin (0.25%, 37°C, 20 min) to obtain oral mucosal cells. These cultured cells showed a colony forming ability of 0.3%, a higher value than common rabbit oral mucosal cells would show. The resulting cells were seeded onto the separately prepared temperature-responsive culture substrate (insert type; CellSeed) at a density of 5×10⁵ cells per insert. On the culture substrate on which to place an insert, mouse 3T3 cells that had lost their proliferation potential by mitomycin C according to a conventional protocol were cultured as feeder cells. The oral mucosal cells were cultured in a DMEM/Ham's F12 medium (3:1; Invitrogen) supplemented with 10% fetal bovine serum (FCS) as well as insulin (5 µg/mL; Invitrogen), cholera toxin (1 nM; Wako Pure Chemical Industries), hydrocortisone (0.4 µg/mL; Wako Pure Chemical Industries), triiodothyronine (2 nM/mL; MP Biomedicals), transferrin (5 µg/mL; Invitrogen), and EGF (10 ng/mL; Invitrogen). As the feeder cells, 2×10⁴ 3T3 cells were used. Twelve days after the culture, the layered oral mucosal cell sheet was detached from the temperature-responsive culture substrate by cooling.

The resulting cell sheet was delipidated by sequential sonications at different chloroform/methanol ratios of 2:1, 1:1 and 1:2 in that order and then subjected to protein digestion with Pronase. Thereafter, the sample was so prepared as to give 80% ethanol and 5% sodium acetate, left to stand at 4°C for 2 hours, and then centrifuged to collect pellets. The collected pellets were subjected to digestion of glycosaminoglycans with Chondroitinase ABC (Proteus vulgaris), Heparinase (Flavobacterium heparinum), Heparitinase (Flavobacterium heparinum), and Hyaluronidase SD (Streptococcus dysgalactiae), and the digested product was purified by glycoblotting, fluorescently labeled by reductive amination, and then separated and analyzed using a ZIC-HILIC column. The zwitterionic column used was ZIC-HILIC (2×150 mm). The stationary phase of the ZIC column consisted of silica particles having N,N-dimethyl,N-methacryloyloxyethyl,N-(3-sulfopropyl)ammonium betaine covalently bonded to the surfaces thereof. Using eluent A (Milli-Q water), eluent B (acetonitrile), and eluent C (200 mM ammonium acetate), a gradient elution was performed under the following conditions: A/B/C=5/90/5 (0 min) → A/B/C=13/82/5 (20 min) → A/B/C=35/60/5 (60 min). The flow rate was fixed at 0.2 mL/min and the column temperature was maintained at 30°C with a column oven. Fluorescence detection was performed at an excitation wavelength of 330 nm and a fluorescent wavelength of 420 nm. The absolute quantity was estimated from the quantity of the internal standard added. The results showed that the content of HA in the cultured cells increased significantly.

### [Example 2]

Using a rabbit (New Zealand White; Charles River Laboratories Japan) as a model and assuming that the model suffers from a loss of the cornea, corneal epithelial cells were prepared from the rabbit. A sclerocorneal fragment was prepared from the ocular tissue and treated with dispase (3.0 U, 37°C, 1 h) and then with trypsin (0.25%, 37°C, 15 min) to obtain corneal epithelial cells. These cultured cells showed a colony forming ability of 3.5%, a higher value than common rabbit oral corneal epithelial would show. The resulting cells were seeded onto the separately prepared temperature-responsive culture substrate (insert type; CellSeed) at a density of 1×10⁵ cells per insert. On the culture substrate on which to place an insert, mouse 3T3 cells that had lost their proliferation potential by mitomycin C according to a conventional protocol were cultured as feeder cells. The corneal epithelial cells were cultured in a DMEM/Ham's F12 medium (3:1; Invitrogen) supplemented with 10% fetal bovine serum (FCS) as well as insulin (5 µg/mL; Invitrogen), cholera toxin (1 nM; Wako Pure Chemical Industries), hydrocortisone (0.4 µg/mL; Wako Pure Chemical Industries), triiodothyronine (2 nM/mL; MP Biomedicals), transferrin (5 µg/mL; Invitrogen), and EGF (10 ng/mL; Invitrogen). As the feeder cells, 2×10⁴ 3T3 cells were used. Twelve days after the culture, the layered corneal epithelial cell sheet was detached from the temperature-responsive culture substrate by cooling.

The resulting cell sheet was delipidated by sequential sonications at different chloroform/methanol ratios of 2:1, 1:1 and 1:2 in that order and then subjected to protein digestion with Pronase. Thereafter, the sample was so prepared as to give 80% ethanol and 5% sodium acetate, left to stand at 4°C for 2 hours, and then centrifuged to collect pellets. The collected pellets were subjected to digestion of glycosaminoglycans with Chondroitinase ABC (Proteus vulgaris), Heparinase (Flavobacterium heparinum), Heparitinase (Flavobacterium heparinum), and Hyaluronidase SD (Streptococcus dysgalactiae), and the digested product was purified by glycoblotting, fluorescently labeled by reductive amination, and then separated and analyzed using a ZIC-HILIC column. The zwitterionic column used was ZIC-HILIC (2×150 mm). The stationary phase of the ZIC column consisted of silica particles having N,N-dimethyl,N-methacryloyloxyethyl,N- (3-sulfopropyl)ammonium betaine covalently bonded to the surfaces thereof. Using eluent A (Milli-Q water), eluent B (acetonitrile), and eluent C (200 mM ammonium acetate), a gradient elution was performed under the following conditions: A/B/C=5/90/5 (0 min) → A/B/C=13/82/5 (20 min) → A/B/C=35/60/5 (60 min). The flow rate was fixed at 0.2 mL/min and the column temperature was maintained at 30°C with a column oven. Fluorescence detection was performed at an excitation wavelength of 330 nm and a fluorescent wavelength of 420 nm. The absolute quantity was estimated from the quantity of the internal standard added. The results showed that the content of HA in the cultured cells increased significantly.

### [Example 3]

One oral mucosal cell sheet as obtained in Example 1 was transplanted to a corneal epithelium stem cell deficiency model which had been prepared according to a conventional protocol by removing stem cells from the limbus of a rabbit eyeball. After 3 weeks, the affected area was observed by the naked eye, and the observation showed that the oral mucosal cell sheet grafted to the eyeball satisfactorily without corneal swelling. The results showed that the transplantation site had an enhanced moisture-retaining property, the state of the affected tissue was improved, and the affected area was healed effectively.

### [Example 4]

One corneal epithelial cell sheet as obtained in Example 2 was transplanted to a corneal epithelium stem cell deficiency model which had been prepared according to a conventional protocol by removing stem cells from the limbus of a rabbit eyeball. After 3 weeks, the affected area was observed by the naked eye, and the observation showed that the corneal epithelial cell sheet grafted to the eyeball satisfactorily without corneal swelling. The results showed that the transplantation site had an enhanced moisture-retaining property, the state of the affected tissue was improved, and the affected area was healed effectively.

### INDUSTRIAL APPLICABILITY

Transplanting the cell sheet prepared according to the present invention onto a recipient's living body enables not only regeneration of a damaged tissue but also improvement of the state of the recipient's tissue by the HA produced from the transplanted cell sheet. In sharp contrast with conventional regenerative medicine techniques that simply enable formation of a damaged tissue in an affected area, this invention can provide a next-generation regenerative medicine technique that enables regeneration of a damaged tissue while improving the environment in an affected area.

## Claims

1. A cell sheet highly producing hyaluronic acid, wherein the cells are any one of cells among oral mucosal cells, limbal epithelial cells, corneal epithelial cells, conjunctival epithelial cells, chondrocytes, synovial cells, epidermal keratinocytes, smooth muscle cells, vascular endothelial cells, fibroblasts and mammary epithelial cells, or a mixture of two or more types thereof,
wherein the cell sheet is obtainable by culturing the cells on a surface of a substrate and harvesting the cells in the form of a sheet,
wherein the cells have stem cells thereof contained therein, and
wherein the stem cell content in the cultured cells is at least 0.5%,
and wherein the cell sheet is detached, as adhered to a carrier, from a cell culture support having a substrate surface coated with a temperature-responsive polymer whose upper or lower critical solution temperature in water is 0 to 80°C.

2. The cell sheet according to claim 1, which is a stack of two or more layers of cell sheet.

3. A method for preparing the cell sheet according to any one of Claims 1-2, wherein cells are placed on a cell culture support having a surface coated with a polymer which changes a hydration ability in a temperature range of 0-80°C, said cells are cultured in a temperature range where the polymer exhibits a weak hydration ability, and then the temperature of a culture solution is changed to a temperature at which the polymer exhibits a strong hydration ability, so that the cultured cells are detached in the form of sheet, with their hyaluronic acid production ability being increased.

4. The method according to Claim 3, wherein the cells are any one type of cells among oral mucosal cells, limbal epithelial cells, corneal epithelial cells, conjunctival epithelial cells, chondrocytes, synovial cells, epidermal keratinocytes, smooth muscle cells, vascular endothelial cells, fibroblasts and mammary epithelial cells, or a mixture of two or more types thereof.

5. The method according to any one of Claims 3 and 4, comprising a step of increasing a stem cell content during the culture.

6. The method according to any one of Claims 3-5, wherein the detached cell sheet is placed on another cell sheet or this step is repeated to thereby prepare a cell sheet stack.

7. The method according to any one of Claims 3-6, wherein the detachment from the cell culture support is achieved without protease treatment.

8. The method according to any one of Claims 3-7, wherein upon completion of the culture, a carrier is adhered to the cultured cells and the cells are detached together with the carrier.

9. The method according to any one of Claims 3-8, wherein the number of the cells to be seeded for culture is in the range from 0.4x10⁶ to 2.5x10⁶ cells/cm².

10. A cell sheet for use in a method of transplantation for providing the recipient's tissue with hyaluronic acid, wherein a cell sheet according to any one of claims 1 to 2, or a cell sheet obtainable by a method according to any one of claims 3 to 9 is transplanted onto a given site in a recipient's living body.

11. The cell sheet for use according to Claim 10, wherein the transplantation site is a skin, an eyeball, a joint, a blood vessel wall tissue, or a cardiac valve tissue in the recipient's living body.

12. The cell sheet for use according to any one of Claims 10 and 11, wherein the transplantation site has blood vessels induced therein beforehand.

13. Use of a cell sheet according to any one of Claims 1-2 or of a cell sheet obtainable by an in vitro method according to any one of Claims 3-9 in a method for producing hyaluronic acid.

## Patentansprüche

1. Zellschicht, welche Hyaluronsäure in hohem Maße herstellt, wobei die Zellen irgendeine der Zellen aus Mundschleimhautzellen, Limbus-Epithelzellen, Hornhautepithelzellen, Bindehautepithelzellen, Knorpelzellen, Synovialzellen, epidermalen Keratinozyten, glatten Muskelzellen, vaskulären Endothelzellen, Fibroblasten und Brustepithelzellen sind, oder eine Mischung von zwei oder mehr Typen davon, wobei die Zellschicht durch Kultivieren der Zellen auf einer Oberfläche eines Substrats und Ernten der Zellen in Form einer Schicht erhältlich ist,
wobei die Zellen Stammzellen davon darin enthalten,
und
wobei der Stammzellengehalt in den kultivierten Zellen mindestens 0.5% beträgt,
und wobei die Zellschicht abgelöst wird, adhärent an einen Carrier, von einem Zellkulturträger, der eine Substratoberfläche hat, die mit einem temperaturempfindlichen Polymer beschichtet ist, dessen obere oder untere kritische Lösungstemperatur in Wasser 0 bis 80°C beträgt.

2. Zellschicht gemäß Anspruch 1, welche ein Stapel von zwei oder mehr Lagen von Zellschicht ist.

3. Verfahren zur Herstellung der Zellschicht gemäß irgendeinem der Ansprüche 1-2, wobei Zellen auf einen Zellkulturträger platziert werden, der eine Oberfläche hat, die mit einem Polymer beschichtet ist, welches in einem Temperaturbereich von 0-80°C eine Hydratisierungsfähigkeit verändert, besagte Zellen in einem Temperaturbereich kultiviert werden, in dem das Polymer eine schwache Hydratisierungsfähigkeit aufweist, und dann die Temperatur einer Kulturlösung zu einer Temperatur verändert wird, bei welcher das Polymer eine starke Hydratisierungsfähigkeit aufweist, so dass die kultivierten Zellen in Form einer Schicht abgelöst werden, wobei ihre Hyaluronsäure-Produktionsfähigkeit erhöht ist.

4. Verfahren gemäß Anspruch 3, wobei die Zellen irgendein Typ von Zellen aus Mundschleimhautzellen, Limbus-Epithelzellen, Hornhautepithelzellen, Bindehautepithelzellen, Knorpelzellen, Synovialzellen, epidermalen Keratinozyten, glatten Muskelzellen, vaskulären Endothelzellen, Fibroblasten und Brustepithelzellen sind, oder eine Mischung von zwei oder mehr Typen davon.

5. Verfahren gemäß irgendeinem der Ansprüche 3 und 4, umfassend einen Schritt zum Erhöhen eines Stammzellengehalts während der Kultur.

6. Verfahren gemäß irgendeinem der Ansprüche 3-5, wobei die abgelöste Zellschicht auf eine andere Zellschicht platziert wird oder dieser Schritt wiederholt wird, um dadurch einen Zellschichtenstapel herzustellen.

7. Verfahren gemäß irgendeinem der Ansprüche 3-6, wobei das Ablösen von dem Zellkulturträger ohne Protease-Behandlung erreicht wird.

8. Verfahren gemäß irgendeinem der Ansprüche 3-7, wobei nach Beendigung der Kultur ein Carrier an die kultivierten Zellen angehaftet wird und die Zellen zusammen mit dem Carrier abgelöst werden.

9. Verfahren gemäß irgendeinem der Ansprüche 3-8, wobei die Anzahl der für die Kultur auszusäenden Zellen in dem Bereich von 0,4x10⁶ bis 2,5x10⁶ Zellen/cm² liegt.

10. Zellschicht zur Verwendung in einem Transplantationsverfahren, um das Gewebe des Empfängers mit Hyaluronsäure zu versorgen, wobei eine Zellschicht gemäß irgendeinem der Ansprüche 1 bis 2, oder eine Zellschicht, erhältlich durch ein Verfahren gemäß irgendeinem der Ansprüche 3 bis 9, auf eine gegebene Stelle in dem lebenden Körper eines Empfängers transplantiert wird.

11. Zellschicht zur Verwendung gemäß Anspruch 10, wobei die Transplantationsstelle eine Haut, ein Augapfel, ein Gelenk, ein Wandgewebe eines Blutgefäßes oder ein Herzklappengewebe in dem lebenden Körper des Empfängers ist.

12. Zellschicht zur Verwendung gemäß irgendeinem der Ansprüche 10 und 11, wobei die Transplantationsstelle vorab induzierte Blutgefäße hat.

13. Verwendung einer Zellschicht gemäß irgendeinem der Ansprüche 1-2 oder einer Zellschicht, erhältlich durch ein in vitro-Verfahren gemäß irgendeinem der Ansprüche 3-9, in einem Verfahren zur Herstellung von Hyaluronsäure.

## Revendications

1. Feuille cellulaire hautement productrices d'acide hyaluronique, dans laquelle les cellules sont une quelconque des cellules parmi des cellules de la muqueuse buccale, des cellules de l'épithélium limbique, des cellules de l'épithélium cornéen, des cellules de l'épithélium conjonctival, des chondrocytes, des cellules synoviales, des kératinocytes épidermiques, des cellules du muscle lisse, des cellules de l'endothélium vasculaire, des fibroblastes et des cellules de l'épithélium mammaire, ou un mélange de deux types ou plus de celles-ci,
dans laquelle la feuille cellulaire peut être obtenue par la mise en culture des cellules sur une surface d'un substrat et la récolte des cellules sous forme d'une feuille,
dans laquelle les cellules ont des cellules souches de celles-ci contenues à l'intérieur de celles-ci, et
dans laquelle la teneur en cellules souches dans les cellules mises en culture est d'au moins 0,5 %,
et dans laquelle la feuille cellulaire est détachée, lorsqu'elle adhère à un vecteur, d'un support cellulaire dont une surface de substrat est recouverte d'un polymère sensible à la température dont la température de solution critique supérieure ou inférieure dans l'eau est de 0 à 80 °C.

2. Feuille cellulaire selon la revendication 1, qui est une pile d'au moins deux couches de feuille cellulaire.

3. Procédé de préparation de la feuille cellulaire selon l'une quelconque des revendications 1 et 2, dans lequel des cellules sont placées sur un support cellulaire ayant une surface recouverte d'un polymère qui modifie une capacité d'hydratation dans une plage de températures de 0 à 80 °C, lesdites cellules sont mises en culture dans une plage de températures où le polymère présente une faible capacité d'hydratation, puis la température d'une solution de culture est modifiée pour atteindre une température à laquelle le polymère présente une capacité d'hydratation élevée, de sorte que les cellules mises en culture sont détachées sous la forme de feuille, leur capacité de production d'acide hyaluronique étant accrue.

4. Procédé selon la revendication 3, dans lequel les cellules sont un type quelconque de cellules parmi des cellules de la muqueuse buccale, des cellules de l'épithélium limbique, des cellules de l'épithélium cornéen, des cellules de l'épithélium conjonctival, des chondrocytes, des cellules synoviales, des kératinocytes épidermiques, des cellules du muscle lisse, des cellules de l'endothélium vasculaire, des fibroblastes et des cellules de l'épithélium mammaire, ou un mélange de deux types ou plus de celles-ci.

5. Procédé selon l'une quelconque des revendications 3 et 4, comprenant une étape d'augmentation d'une teneur en cellules souches pendant la culture.

6. Procédé selon l'une quelconque des revendications 3 à 5, dans lequel la feuille cellulaire détachée est placée sur une autre feuille cellulaire ou cette étape est répétée pour préparer ainsi une pile de feuilles cellulaire.

7. Procédé selon l'une quelconque des revendications 3 à 6, dans lequel le détachement du support cellulaire est obtenu sans traitement protéasique.

8. Procédé selon l'une quelconque des revendications 3 à 7, dans lequel au terme de la culture, un vecteur adhère aux cellules en culture et les cellules sont détachées ensemble avec le vecteur.

9. Procédé selon l'une quelconque des revendications 3 à 8, dans lequel le nombre de cellules à ensemencer est dans la plage de 0,4x10⁶ à 2,5x10⁶ cellules/cm².

10. Feuille cellulaire à utiliser dans un procédé de transplantation pour fournir au tissu du receveur de l'acide hyaluronique, dans laquelle une feuille cellulaire selon l'une quelconque des revendications 1 et 2, ou une feuille cellulaire pouvant être obtenue par un procédé selon l'une quelconque des revendications 3 à 9 est transplantée sur un site donné dans le corps d'un receveur vivant.

11. Feuille cellulaire selon la revendication 10, dans laquelle le site de transplantation est une peau, un globe oculaire, une articulation, un tissu de paroi de vaisseau sanguin ou un tissu de valvule cardiaque dans le corps du receveur vivant.

12. Feuille cellulaire à utiliser selon l'une quelconque des revendications 10 et 11, dans laquelle le site de transplantation a des vaisseaux sanguins induits à l'intérieur de celui-ci au préalable.

13. Utilisation d'une feuille cellulaire selon l'une quelconque des revendications 1 et 2 ou d'une feuille cellulaire pouvant être obtenue par un procédé in vitro selon l'une quelconque des revendications 3 à 9 dans un procédé de production d'acide hyaluronique.
